# EUROPEAN PATENT APPLICATION

(11) **EP 4 740 838 A1**
(43) Date of publication of application: **13.05.2026**
(21) Application number: 23959162.1
(22) Date of filing: 22.11.2023
(51) Int. Cl.: A61B 5/00

(54) **MONITORING AUXILIARY APPARATUS AND MONITORING SYSTEM**

(71) Applicant: Shenzhen Mindray Bio-Medical Electronics Co., Ltd., Shenzhen, Guangdong 518057 (CN)
(72) Inventor: LI, Xinsheng, Shenzhen, Guangdong 518057 (CN); CEN, Jian, Shenzhen, Guangdong 518057 (CN); XU, Li, Shenzhen, Guangdong 518057 (CN); LIU, Qiling, Shenzhen, Guangdong 518057 (CN); ZHANG, Kui, Shenzhen, Guangdong 518057 (CN); QING, Lei, Shenzhen, Guangdong 518057 (CN); YIN, Jun, Shenzhen, Guangdong 518057 (CN); PAN, Ruiling, Shenzhen, Guangdong 518057 (CN); NING, Li, Shenzhen, Guangdong 518057 (CN); LI, Ming, Shenzhen, Guangdong 518057 (CN); CHEN, Zhanggen, Shenzhen, Guangdong 518057 (CN)
(74) Representative: KIPA AB
(86) International application number: PCT/CN2023/133339
(87) International publication number: WO 2025/107188

(57) **Abstract**

This disclosure provides a monitoring auxiliary device and a monitoring system. The monitoring auxiliary device is in wired connection with multiple types of sensors to obtain physiological parameter signals detected by the sensors. The monitoring auxiliary device outputs monitoring-related data in a wired manner. When not in wired connection with the monitor, the monitoring-related data is transmitted to a monitor through a wireless communication module. The monitoring auxiliary device summarizes data from various sensors and outputs said data to the monitor in a wired manner or a wireless manner, requiring very few cables to connect with the monitor, thereby reducing a workload of medical staff plugging and unplugging cables and maintaining a clean surface of patient support.

## Description

### TECHNICAL FIELD

The disclosure relates to a technical field of medication, and in particular, relates to a monitoring auxiliary device and a monitoring system.

### BACKGROUND

Patients need continuous monitoring in multiple scenarios in a hospital, and requirements for monitoring vary in different environments. A traditional monitor typically has multiple interfaces, wherein each interface is connected with a sensor via a cable to collect physiological parameters of patient. The monitor only provides effective monitoring for a patient lying in a patient support. When a patient moves, such as such as getting out of patient support and being transported, many cables need to be plugged and unplugged, which is complex to operate and affects a work efficiency of medical staff. The monitoring is interrupted for a relatively long time.

When a patient is on a patient support, traditional monitor cables are long and disorganized, requiring a lot of maintenance work for medical staff.

### SUMMARY

This disclosure mainly provides a monitoring auxiliary device and a monitoring system, aiming to improve a work efficiency of medical staff in monitoring scenario(s).

An embodiment provides a monitoring auxiliary device including: one or more first interfaces, a second interface, a first wireless communication module and a second wireless communication module;
the one or more first interfaces are in wired connection with multiple types of sensors, so as to obtain physiological parameter signals detected by the sensors;
the second interface is connected with a monitor via a cable, so as to output monitoring-related information in a wired manner; wherein the monitoring-related information includes the physiological parameter signals detected by the sensors, and/or physiological parameter data that are obtained based on processing the physiological parameter signals detected by the sensors;
the first wireless communication module is configured to output the monitoring-related information in a wireless manner; the second wireless communication module is also configured to output the monitoring-related information in a wireless manner; wherein the first wireless communication module and the second wireless communication module use different wireless communication manners;
when the second interface is not in wired connection with the monitor, the first wireless communication module and the second wireless communication module simultaneously transmit the monitoring-related information to the monitor.

An embodiment provides a monitoring auxiliary device, including: a first interface, a second interface and one or more wireless communication modules;
the first interface is in wired connection with a sensor, so as to obtain a physiological parameter signal detected by the sensor;
the one or more wireless communication modules are configured to output monitoring-related information in a wireless manner; wherein the monitoring-related information includes the physiological parameter signal detected by the sensor, and/or physiological parameter data that is obtained based on processing the physiological parameter signal;
when the second interface is in wired connection with a monitor, the second interface outputs the monitoring-related information to the monitor in a wired manner; wherein at least one of the one or more wireless communication modules transmits the monitoring-related information to the monitor, while the second interface outputs the monitoring-related information in a wired manner; or
when the second interface is connected with the monitor via a cable, the second interface outputs the monitoring-related information to the monitor in a wired manner; wherein at least one of the one or more wireless communication modules maintains a wireless communication connection with the monitor, while the second interface outputs the monitoring-related information in a wired manner.

An embodiment provides a monitoring auxiliary device, including: a first interface, a second interface, a first wireless communication module, and a second wireless communication module;
the first interface is in wired connection with a sensor, so as to obtain a physiological parameter signal detected by the sensor;
the second interface is connected with a monitor via a cable, so as to output monitoring-related information in a wired manner; wherein the monitoring-related information includes the physiological parameter signal detected by the sensor, and/or physiological parameter data that is obtained based on processing the physiological parameter signal;
the first wireless communication module is configured to output the monitoring-related information in a wireless manner;
the second wireless communication module is also configured to output the monitoring-related information in a wireless manner; wherein the first wireless communication module and the second wireless communication module use different wireless communication manners;
when the second interface is not in wired connection with the monitor and a preset condition is satisfied, the first wireless communication module transmits the monitoring-related information to the monitor;
when the second interface is not in wired connection with the monitor and the preset condition is not satisfied, the second wireless communication module transmits the monitoring-related information to the monitor, or the first wireless communication module and the second wireless communication module simultaneously transmit the monitoring-related information to the monitor;
wherein the preset condition includes: a wireless communication connection between the first wireless communication module and the monitor is reliable, and/or the monitoring auxiliary device is located within a preset range.

An embodiment provides a monitoring auxiliary device adapted to a holding device, wherein the holding device is detachably mounted on a patient support for holding the monitoring auxiliary device on the patient support; wherein the monitoring auxiliary device includes:
a first interface, which is in wired connection with a sensor, so as to obtain a physiological parameter signal detected by the sensor;
a second interface, which is connected with a monitor via a cable and outputs monitoring-related information in a wired manner; wherein the monitoring-related information includes the physiological parameter signal detected by the sensor, and/or physiological parameter data that is obtained based on processing the physiological parameter signal;
a wireless communication module, which is configured to output the monitoring-related information in a wireless manner;
when the monitoring auxiliary device is held by the holding device, the monitoring-related information that is outputted by the second interface is transmitted to the monitor via the holding device;
when the monitoring auxiliary device is not held by the holding device and the second interface does not output the monitoring-related information, the wireless communication module transmits the monitoring-related information to the monitor.

An embodiment provides a monitoring auxiliary device adapted to a holding device, wherein the holding device is detachably mounted on a patient support for holding the monitoring auxiliary device on the patient support; wherein the monitoring auxiliary device includes:
a first interface, which is in wired connection with a sensor, so as to obtain a physiological parameter signal detected by the sensor;
a second interface, which is connected with a monitor via a cable and outputs monitoring-related information in a wired manner; wherein the monitoring-related information includes the physiological parameter signal detected by the sensor, and/or physiological parameter data that is obtained based on processing the physiological parameter signal;
when the monitoring auxiliary device is held by the holding device, the monitoring-related information that is outputted by the second interface is transmitted to the monitor via the holding device.

An embodiment provides a monitoring system including: multiple sensors, a monitoring auxiliary device, a transport monitor and a main monitor;
the monitoring auxiliary device is in wired connection with the multiple sensors, so as to obtain physiological parameter signals detected by the multiple sensors;
the monitoring auxiliary device is configured to output monitoring-related information to the transport monitor in a wired manner and/or a wireless manner, when the monitoring auxiliary device is in wired connection with the transport monitor; the monitoring auxiliary device is further configured to output the monitoring-related information to the transport monitor in a wireless manner, when the monitoring auxiliary device is not in wired connection with the transport monitor; wherein the monitoring-related information includes the physiological parameter signals detected by the sensors, and/or physiological parameter data that are obtained based on processing the physiological parameter signals;
the transport monitor is configured to receive the monitoring-related information that is outputted by the monitoring auxiliary device, and transmit to the main monitor the monitoring-related information or result(s) calculated based on the monitoring-related information.

An embodiment provides a monitoring system, including: multiple sensors, a monitoring auxiliary device, a transport monitor, and a main monitor;
the monitoring auxiliary device is in wired connection with the multiple sensors, so as to obtain physiological parameter signals detected by the multiple sensors;
the monitoring auxiliary device is configured to output monitoring-related information to the transport monitor in a wired manner; wherein the monitoring-related information includes the physiological parameter signals detected by the sensors, and/or physiological parameter data that are obtained based on processing the physiological parameter signals;
the transport monitor is configured to receive the monitoring-related information that is outputted by the monitoring auxiliary device, and output to the main monitor the monitoring-related information or result(s) calculated based on the monitoring-related information.

According to above embodiments of monitoring auxiliary device and monitoring system, a monitoring auxiliary device is in wired connection with multiple types of sensors to obtain physiological parameter signals detected by the sensors; wherein the monitoring auxiliary device is capable of outputting monitoring-related information in a wired manner. When no wired connection exists between the monitor and the monitoring auxiliary device, the monitoring auxiliary device is capable of transmitting the monitoring-related information to the monitor via a wireless communication module. It can be seen that the monitoring auxiliary device aggregates data from various sensors and outputs said data to the monitor in a wired manner or a wireless manner, requiring very few cables to be connected with the monitor, thereby reducing a workload of medical staff for plugging and unplugging cables and maintaining a clean bed surface.

### BRIEF DESCRIPTION OF THE DRAWINGS

FIG. 1 is a structural block diagram for an embodiment of a monitoring system provided by this disclosure.
FIG. 2 is a schematic diagram for an embodiment of a monitoring system provided by this disclosure.
FIG. 3 is a flowchart for an embodiment of a method for collecting monitoring-related information provided by this disclosure.
FIG. 4 is a flowchart for an embodiment of a monitoring auxiliary device in a monitoring system provided by this disclosure.
FIG. 5 is an axonometric view for an embodiment of a monitoring auxiliary device and a holding device in a monitoring system provided by this disclosure.
FIG. 6 is a schematic diagram for an embodiment of an electrical connection between a monitoring auxiliary device and a monitor in a monitoring system provided by this disclosure.
FIG. 7 is a structural block diagram for an embodiment of a monitoring system provided by this disclosure.
FIG. 8 is a schematic diagram for a patient carrying a monitoring auxiliary device and multiple sensors to get out of patient support in a monitoring system provided by this disclosure.
FIG. 9 is a schematic diagram for a transportation scenario in a monitoring system provided by this disclosure.
FIG. 10 is a flowchart for an embodiment of a method for collecting monitoring-related information provided by this disclosure.
FIG. 11 is a flowchart for an embodiment of a method for collecting monitoring-related information provided by this disclosure.
FIG. 12 is a structural block diagram for an embodiment of a monitoring system provided by this disclosure.
FIG. 13 is a flowchart for an embodiment of a method for collecting monitoring-related information provided by this disclosure.

### DESCRIPTION OF THE PREFERRED EMBODIMENTS

This disclosure will be further described in detail below through specific embodiments in combination with the accompanying drawings. In different embodiments, similar elements adopt associated similar reference numbers. In the following embodiments, many details are described so as to make this disclosure better understood. However, one skilled in the art can easily recognize that some of the features can be omitted in different cases, or can be replaced by other elements, materials and methods. In some cases, some operations related to this disclosure are not shown or described in the description, so as to avoid a core part of this disclosure being inundated by too many descriptions. For one skilled in the art, it is not necessary to describe these related operations in detail, as they can fully understand relevant operations according to the description in this disclosure and the general technical knowledge in the art.

In addition, the features, operations, or characteristics described in the description may be combined in any appropriate manner to form various embodiments. At the same time, steps or actions in method description can also be exchanged or adjusted in order in a manner obvious to one skilled in the art. Therefore, various sequences in the description and the drawings are only for a purpose of clearly describing a certain embodiment, and do not mean that they are necessary sequences. Unless it is otherwise specified that a sequence must be followed.

Serial numbers assigned to components in this disclosure, such as "first", "second", etc., are only used to distinguish objects described and do not have any order or technical meaning. In addition, terms "connection", and "linkage" mentioned in this disclosure include direct and indirect connection (linkage), unless otherwise specified.

This disclosure is arranged with a monitoring auxiliary device between sensor(s) and a monitor, which summarizes cable(s) of each sensor, simplify a layout and a length of cable(s) on a patient support, and strive to improve a convenience degree for a monitoring scenario, such as when a patient moving on the patient support, getting out of the patient support, and being transported, and reduce a complexity of a nurse operation. Below are some implementation examples for detailed explanation.

### First embodiment

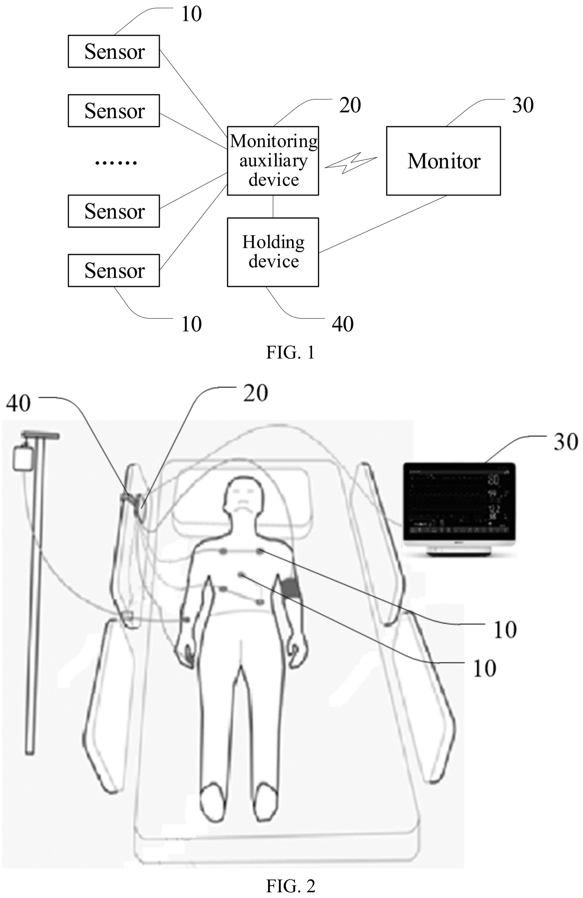

This disclosure adds a monitoring auxiliary device and a holding device 40 to a traditional monitoring system, as shown in FIGS. 1 and 2. The monitoring system includes multiple types of sensors 10, a monitoring auxiliary device 20, a holding device 40, and a monitor 30.

The sensors 10 are configured to detect physiological parameter signals of a patient. Among the multiple types of sensors 10 in the monitoring system, different types of sensors 10 are used to detect different physiological parameter signals. For example, these different physiological parameter signals include various types of signals, such as a blood oxygen signal, an electrocardiogram signal, a body temperature signal, a respiratory signal, and a blood pressure signal. The multiple types of sensors 10 can include various types of sensors, such as a temperature sensor, a lead, a blood oxygen sensor, a pressure sensor, and a respiratory rate sensor. For example, the multiple types of sensors 10 include a temperature sensor to detect a body temperature signal of a patient. For example, the multiple types of sensors 10 include a lead to detect an electrocardiogram signal of a patient. For example, the multiple types of sensors 10 include a blood oxygen sensor to detect a blood oxygen signal of a patient, so as to obtain blood oxygen saturation data. For example, the multiple types of sensors 10 include a pressure sensor to detect a blood pressure signal of a patient. For example, the multiple types of sensors 10 include a respiratory rate sensor to detect a respiratory rate of a patient. For example, the multiple types of sensors 10 may include other sensors.

In this embodiment, the monitoring system includes multiple different types of sensors, one of which is a pressure sensor used to detect a blood pressure signal. The monitoring auxiliary device 20 also includes a gas pump, which is built into a housing of the monitoring auxiliary device 20, and configured to press a cuff of the pressure sensor to detect a blood pressure signal, thereby performing non-invasive blood pressure detection. The gas pump is connected with the cuff through a trachea, and the gas pump is arranged inside the monitoring auxiliary device 20, which shortens a length of the trachea and facilitates storage and organization.

The monitoring auxiliary device 20 is in wired connection with multiple types of sensors 10, that is, in wired connection with multiple different sensors, so as to obtain physiological parameter signals detected by the sensors 10. That is to say, the various physiological parameter signals of a patient collected by multiple sensors 10 are summarized into the monitoring auxiliary device 20 through cables.

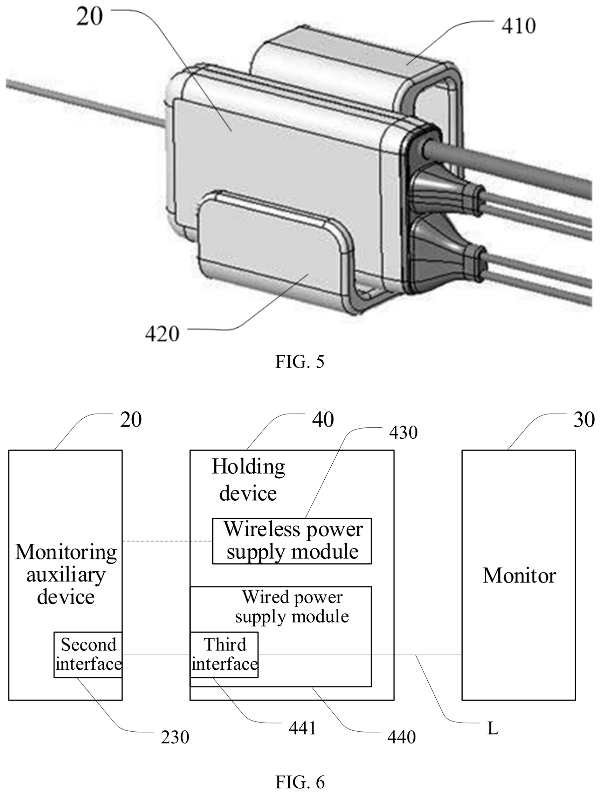

The monitoring auxiliary device 20 is adapted to the holding device 40. The holding device 40 is detachably mounted on the patient support for holding the monitoring auxiliary device 20, so as to hold the monitoring auxiliary device on the patient support. As shown in FIG. 5, the holding device 40 includes a fixing portion 410 and a mounting portion 420. Wherein, the fixing portion 410 is detachably mounted on the patient support. For example, the fixing portion 410 is a hook structure that can be hung on a guardrail of the patient support and is easily detachable. For example, the fixing portion 410 is a clamping structure that can be clamped on a guardrail of the patient support and is easily detachable. For example, the fixing portion 410 includes both a hook structure and a clamping structure, and the fixing portion 410 is hung on a guardrail through the hook structure and clamps by the clamping structure, so as to adapt to guardrails of different thicknesses. The mounting portion 420 is configured to mount the monitoring auxiliary device 20, which may include a receiving slot that can stably accommodate the monitoring auxiliary device 20.

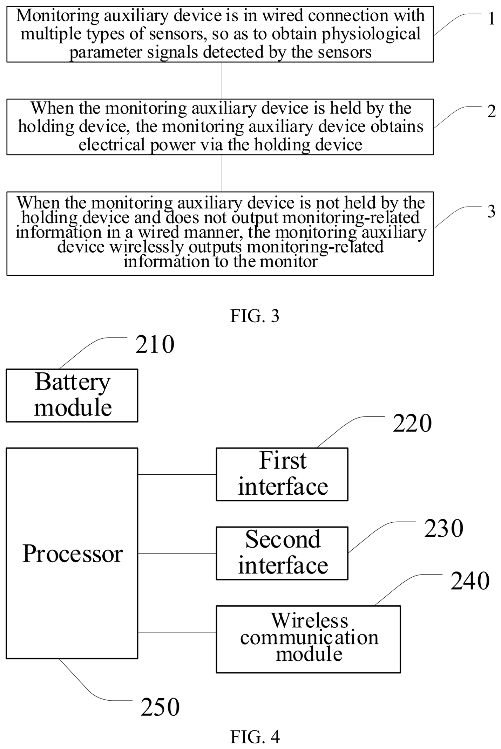

As shown in FIG. 4, in an embodiment, the monitoring auxiliary device 20 includes a battery module 210, a first interface 220, a second interface 230, a wireless communication module 240, and a processor 250.

The first interface 220 is in wired connection with the sensors 10, so as to obtain physiological parameter signals detected by the sensors 10. For example, the monitoring auxiliary device 20 has one first interface 220, which is capable of connecting with multiple different types of sensors 10 through cable(s). For example, the monitoring auxiliary device 20 has multiple first interfaces 220, each first interface is connected with a type of sensor 10 via a cable. A number and type of first interfaces 220 of the monitoring auxiliary device 20 can satisfy connection requirement(s) of all sensors of the monitor. Of course, some first interface(s) 220 can also be connected with one sensor 10 through cables, while other first interface(s) 220 can be connected with multiple sensors 10 through cable(s). In summary, the monitoring auxiliary device 20 can be in wired connection with multiple different types of sensors 10 through one or more first interfaces 220.

It is worth noting that in this scheme, the first interface can be manufactured by "interface merging", which means merging interfaces corresponding to multiple types of sensors. That is, one first interface can receive parameters transmitted by more than one type of sensors, such as a blood oxygen sensor and an electrocardiogram sensor, which sensors are connected with the same first interface. Ultimately, the number of the first interfaces in this scheme may be less than the number of the type of the sensors.

The second interface 230 is configured to output monitoring-related information in a wired manner. For example, the second interface 230 is connected with the monitor 30 via a cable, so as to output monitoring-related information to the monitor 30 in a wired manner, as well as obtain electrical power from an external power source for the monitoring auxiliary device 20 to use. It can be seen that in some embodiments, a same second interface 230 can have two different functions, namely, to simultaneously achieve data transmission and power supply. Of course, in other embodiments, there may be two types of second interfaces 230, one second interface is configured to output monitoring-related information in a wired manner, the other second interface is configured to obtain electrical power from an external power source for the monitoring auxiliary device 20 to use. In this case, the monitoring auxiliary device 20 uses different interfaces for wired data transmission and electrical power supply. The monitoring-related information includes physiological parameter signals detected by the sensor 10, and/or physiological parameter data that are obtained based on processing the physiological parameter signals detected by the sensors 10.

The wireless communication module 240 is configured to output monitoring-related information in a wireless manner. For example, the wireless communication module 240 can use WMTS module (wireless medical telemetry service module), WIFI module, Bluetooth module, etc. The wireless communication module 240 can be wirelessly manner pre-paired with the monitor 30, and then output monitoring-related information to the monitor 30 in a wireless manner.

The battery module 210 is configured to supply electrical power to the monitoring auxiliary device 20, when no external power source exists. The battery module 210 can include rechargeable batteries or battery compartments.

The processor 250 is configured to control the monitoring auxiliary device 20 to achieve various functions of the monitoring auxiliary device 20. For example, the processor 250 is capable of outputting the physiological parameter signals detected by the sensor 10 through the second interface 230 and/or the wireless communication module 240. The processor 250 is also capable of processing the physiological parameter signals detected by the sensor 10 to obtain physiological parameter data, such as processing the blood oxygen signal to obtain blood oxygen saturation, processing the electrocardiogram signal to obtain electrocardiogram waveform, etc., and then outputting the physiological parameter data through the second interface 230 and/or the wireless communication module 240.

When the monitoring auxiliary device 20 is held by the holding device 40, the monitoring auxiliary device 20 obtains electrical power via the holding device 40. There are various specific ways to obtain electrical power, and two of them are listed below for explanation.

One power supply method is wireless power supply, wherein the monitoring auxiliary device 20 also includes a wireless charge-receiving module. The wireless charge-receiving module is configured to receive electrical power from an external device in a wireless manner, for example, the wireless charge-receiving module includes a wireless charge-receiving coil that can receive electrical power emitted by an adapted wireless charge-transmitting coil.

Correspondingly, as shown in FIG. 6, the holding device 40 may also include a wireless power supply module 430, which is adapted to the wireless charge-receiving module and configured to supply electrical power to the monitoring auxiliary device 20 in a wireless manner. For example, the wireless power supply module 430 includes a wireless charge-transmitting coil that can transmit electrical power to the wireless charge-receiving coil of the monitoring auxiliary device 20 in a wireless manner.

Thus, the monitoring auxiliary device 20 obtains electrical power from the holding device 40 through its wireless charge-receiving module, and uses such electrical power. When the monitoring auxiliary device 20 is held by the holding device 40, a position of the wireless power supply module 430 corresponds to a position of the wireless charge-receiving module, so that the holding device 40 can efficiently charge the monitoring auxiliary device 20 in a wireless manner. The electrical power received by the wireless charge-receiving module can be supplied to various functional modules of the monitoring auxiliary device 20, and can also be supplied to charge the battery in the battery module 210. It can be seen that holding the monitoring auxiliary device 20 by the holding device 40 enables wireless charging, which is very convenient. Moreover, the wireless charging method eliminates a need for cables and interfaces, preventing accumulation of dirt and making it easy to clean and disinfect.

Another power supply method is wired power supply. The monitoring auxiliary device 20 can be connected with the holding device 40 through the second interface 230, and electrical power can be obtained from the holding device 40 through the second interface 230 for the monitoring auxiliary device 20 to use. That is, the holding device 40 supplies electrical power to the monitoring auxiliary device 20 in a wired manner. For example, as shown in FIG. 6, the holding device 40 includes a wired power supply module 440. One end of the wired power supply module 440 is connected with the monitor 30 via a cable, so as to obtain electrical power from the monitor 30. The other end of the wired power supply module 440 is in electrical connection with the monitoring auxiliary device 20. In this embodiment, the other end of the wired power supply module 440 is specifically in electrical connection with the second interface 230. The wired power supply module 440 is configured to provide electrical power from the monitor 30 to the monitoring auxiliary device 20. For example, the wired power supply module 440 can mainly consist of interfaces and transmission lines, serving as a circuit bridge between the second interface 230 and the monitor 30, allowing voltage and current output from the monitor 30 to be transmitted through the wired power supply module 440 to the second interface 230. For example, the wired power supply module 440 can process voltage and current outputted by the monitor 30 and then output the processed voltage and current to the second interface 230. Corresponding processing can be one or more of voltage reduction, boost, filtering, current limiting, and energy storage. This embodiment is illustrated using a previous example, as shown in FIG. 6. In this embodiment, the wired power supply module 440 includes a third interface 441, which is adapted to the second interface 230. A rear end of the third interface 441 is in wired connection with the monitor 30 via a cable, and a front end of the third interface 441 is connected (such as docked) with the second interface 230. The second interface 230 and the third interface 441 can use various types of known interfaces, such as pluggable interfaces, contact interfaces, etc., so that the two interfaces can be directly connected and disconnected in a pluggable manner. In this embodiment, the two interfaces use Pogopin interfaces (spring interfaces), which are easy to clean and disinfect during long-term use, and are also easy to connect and disconnect. Furthermore, the Pogopin interfaces also have magnets, which achieve positioning and connection of the second interface 230 and the third interface 441 through magnetic attraction. That is, in this embodiment, the second interface 230 and the third interface 441 use magnetic attraction contact interfaces, so that when the monitoring auxiliary device 20 is held by the holding device 40, the second interface 230 and the third interface 441 are in magnetic attraction connection with each other, which is easy to operate. Of course, in some embodiments, the second interface 230 and the third interface 441 can also be connected by cables, and these two interfaces can use a same type of interfaces or different types of interfaces. Electrical connection can also be achieved through adapter cables.

The wired power supply in this embodiment is as follows. When the monitoring auxiliary device 20 is held by the holding device 40, and the second interface 230 is connected with the third interface 441, the second interface 230 obtains electrical power from the monitor 30 via a circuit that is formed by connecting the third interface 441 and a cable L. That is, for the monitoring auxiliary device 20 or the processor, when the monitoring auxiliary device 20 is held by the holding device 40 and the second interface 230 is connected with the third interface 441, a power supply circuit formed by connecting the second interface 230, the third interface 441, and the cable L (the cable between the third interface 441 and the monitor 30) obtains electrical power from the monitor 30 and supply said electrical power to the monitoring auxiliary device 20. Voltage and current provided by monitor 30 only pass through the holding device 40, and the holding device 40 only serves as a conductor. Therefore, a structure of the holding device 40 can be simple and easy to implement.

Of course, in some embodiments, the above two power supply methods can coexist, that is, the monitoring auxiliary device 20 can obtain electrical power from the holding device 40 in both a wireless manner and a wired manner, even if a connection between the second interface 230 and the holding device 40 is loose, the power supply of the monitoring auxiliary device 20 is not affected.

When the monitoring auxiliary device 20 is held by the holding device 40, monitoring-related information, which is outputted by the monitoring auxiliary device 20 in a wired manner, is transmitted to the monitor 30 via the holding device 40, and the monitoring-related information can also be outputted to the monitor 30 through a wireless manner. The monitoring-related information may include: the physiological parameter signals mentioned above, and/or the physiological parameter data mentioned above. That is to say, the physiological parameter signals detected by each sensor are either transmitted to the monitor 30 by the monitoring auxiliary device 20, or processed into physiological parameter data and transmitted to the monitor 30 by the monitoring auxiliary device 20. Which physiological parameter signals need to be processed into physiological parameter data, and which do not, can be pre-set.

Specifically, if the monitoring auxiliary device 20 obtains electrical power from the holding device 40 in a wireless manner as described above, the processor 250 can sense whether a wireless power supply module 430 exists through the wireless charge-receiving module, so as to determine that the monitoring auxiliary device 20 has been held by the holding device 40 and output monitoring-related information in a wired manner and/or a wireless manner. If the monitoring auxiliary device 20 obtains electrical power from the holding device 40 in a wired manner described above, the processor 250 can detect through the second interface 230 that whether the second interface 230 is connected with the holding device 40 (specifically, connected with the third interface 441), so as to determine that the monitoring auxiliary device 20 has been held by the holding device 40, and then output monitoring-related information in a wired manner and/or a wireless manner.

Specifically, if the monitoring auxiliary device 20 obtains electrical power from the holding device 40 in a wireless manner as described above, the processor 250 can sense whether a wireless power supply module 430 exists through the wireless charge-receiving module, so as to determine that the monitoring auxiliary device 20 has been held by the holding device 40 and output monitoring-related information in a wired manner and/or a wireless manner. If the monitoring auxiliary device 20 obtains electrical power from the holding device 40 in a wired manner described above, the processor 250 can detect through the second interface 230 that whether the second interface 230 is connected with the holding device 40 (specifically, connected with the third interface 441), so as to determine that the monitoring auxiliary device 20 has been held by the holding device 40, and then output monitoring-related information in a wired manner and/or a wireless manner.

The monitoring auxiliary device 20 outputting monitoring-related information in a wired manner, can be that, for example, the processor 250 outputs monitoring-related information through the second interface 230, and the monitoring-related information is transmitted to the monitor 30 after passing through the holding device 40. The monitoring-related information being transmitted to the monitor 30 after passing through the holding device 40, can be that the monitoring-related information is transmitted to the monitor 30 through a wired line of the holding device 40. For example, the monitoring-related information outputted by the monitoring auxiliary device 20 is transmitted to the monitor 30 through the wired power supply module 440 and the cable L. Of course, a cable can also be directly plugged into the second interface of the monitoring auxiliary device 20, through which cable monitoring-related information can be transmitted directly to the monitor without an intervention of the holding device 40. In addition, the monitoring auxiliary device 20 and the monitor 30 share a same cable for transmitting electrical power and monitoring-related information, this is not only simple and easy to manage, but also easy to connect and disconnect. The existing remote monitoring usually involves wearable terminals worn on the human body to monitor physiological parameters and wirelessly transmitting the monitored physiological parameters to a monitoring device, wherein the wearable terminals usually need to be removed and charged, and cannot continue to detect physiological parameters during charging, as they cannot be worn on the human body. The monitoring auxiliary device 20 of this disclosure is different from the wearable terminals. It can not only be charged while obtaining physiological parameter signals, achieving uninterrupted operation, but also converge multiple sensor cables to the monitoring auxiliary device 20, which can transmit electrical power and monitoring-related information through a same cable, simplifying a cable structure and making it very convenient for medical staff to manage and maintain the cables on the patient support. In this embodiment, specifically when the monitoring auxiliary device 20 is held by the holding device 40 and the second interface 230 is connected with the third interface 441, the monitoring-related information that is outputted by the second interface 230 is transmitted to the monitor 30 via the circuit formed by connecting the third interface 441 and the cable. That is, for the monitoring auxiliary device 20 or the processor, when the monitoring auxiliary device 20 is held by the holding device 40 and the second interface 230 is connected with the third interface 441, the monitoring-related information is transmitted to the monitor 30 through a power supply circuit. In this case, the holding device 40 can only serve as a transmission channel for electrical power and monitoring-related information. Of course, in other embodiments, the holding device 40 may also transmit the monitoring-related information that is outputted by the second interface 230 to the monitor 30 in a wireless manner.

The monitoring auxiliary device 20 outputting monitoring-related information in a wireless manner, can be that for example, the processor 250 outputs monitoring-related information through the wireless communication module 240. The monitoring-related information can be transmitted to the monitor 30 via the holding device 40 or directly transmitted to the monitor 30. This embodiment takes the latter as an example for explanation. The monitoring-related information is transmitted to the monitor 30 after passing through the holding device 40 as follows. A wireless communication connection is established between the wireless communication module of the monitoring auxiliary device 20 and the wireless communication module of the holding device 40. The wireless communication module of the monitoring auxiliary device 20 wirelessly transmits the monitoring-related information to the holding device 40, so as to enable the holding device 40 to transmit the monitoring-related information to the monitor 30. For example, the holding device 40 wirelessly transmits the monitoring-related information to the monitor 30 through its wireless communication module, or transmits the monitoring-related information to the monitor 30 in a wired manner via a cable.

In order to improve a reliability of transmitting monitoring-related information to the monitor 30, when the monitoring auxiliary device 20 is held by the holding device 40, the monitoring-related information that is outputted by the second interface 230 is transmitted to the monitor 30 via the holding device 40; and the wireless communication module 240 of the monitoring auxiliary device 20 can transmit the monitoring-related information to the monitor 30 at the same time. For example, after the processor 250 determines that the monitoring auxiliary device 20 is held by the holding device 40, the processor 250 outputs monitoring-related information through the second interface 230 in a wired manner, and outputs monitoring-related information through the wireless communication module 240 in a wireless manner.

From the above content, it can be seen that the monitoring auxiliary device 20 mainly functions to gather the physiological parameter signals collected by various sensors and output them to the monitor 30 together. The monitoring auxiliary device 20 may not have a function of calculating and observing monitoring signals, so that the monitoring auxiliary device 20 may not have a display screen. The function of monitoring a patient based on monitoring-related information is entrusted to the monitor 30.

After receiving monitoring-related information, the monitor 30 can directly display it, or process at least part of physiological parameter signals into physiological parameter data and display the physiological parameter data. The monitor 30 can also process at least part of received monitoring-related information to obtain monitoring result(s). This embodiment takes an example of processing all received monitoring-related information to obtain a monitoring result. The monitoring result can include whether physiological parameter(s) exceed(s) preset range(s), as well as corresponding prompt or alarm information. For example, the monitor 30 determines whether physiological parameter(s) exceed(s) preset range(s) based on physiological parameter signal(s) or physiological parameter(s) in the physiological parameter data. If yes, the physiological parameter(s) is(are) indicated to be abnormal, the monitor 30 generates corresponding prompt or alarm information, and displays the prompt or alarm information. The monitor 30 uses conventional methods for monitoring based on data collected by various sensors, which will not be elaborated here.

The monitor 30 can receive monitoring-related information in both wired manner and wireless manner. The monitor 30 can mainly use the monitoring-related information received in a wired manner for monitoring, and use the monitoring-related information received in a wireless manner for monitoring when the monitoring-related information cannot be received in the wired manner. The monitor 30 can mainly use the monitoring-related information which first arrives at the monitor, no matter which manner said monitoring-related information is employed. The monitor 30 can be a bedside monitor or a central monitoring station, and conventional monitors can be used without limitation in this disclosure.

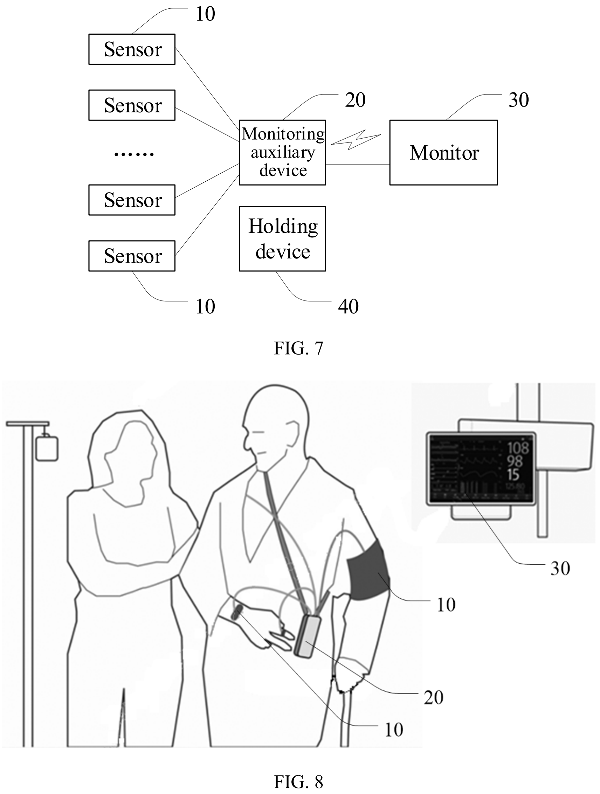

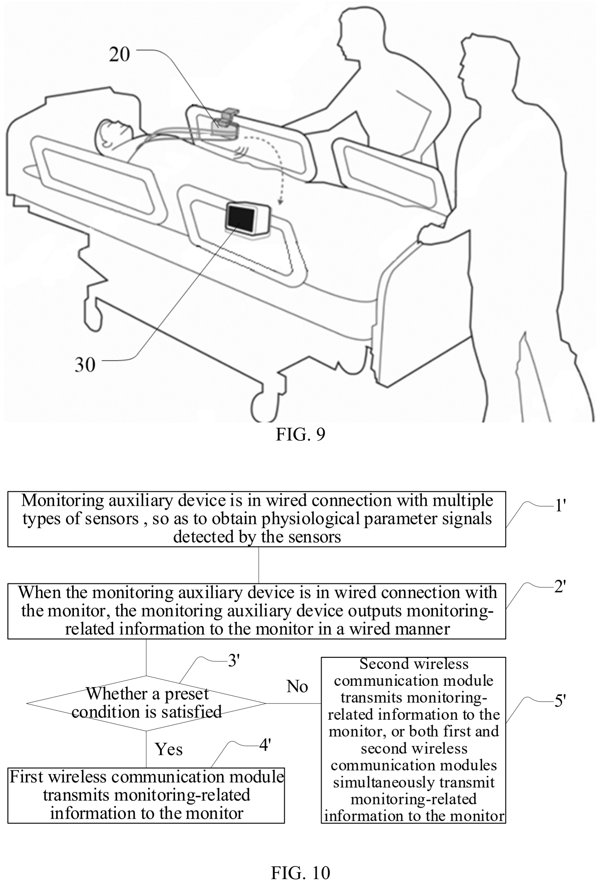

When the monitoring auxiliary device 20 is not held by the holding device 40 and does not output monitoring-related information in a wired manner, such as if the second interface is neither in wired connection with the holding device 40 nor in wired connection with the monitor 30, the monitoring auxiliary device 20 outputs monitoring-related information to the monitor 30 through a wireless manner. That is, when the monitoring auxiliary device 20 is not held by the holding device 40 and the processor 250 does not output monitoring-related information through the second interface 230, the monitoring-related information is directly transmitted to the monitor 30 through the wireless communication module 240. For example, if the processor 250 does not detect that a wireless power supply module 430 exists through the wireless charge-receiving module, it determines that the monitoring auxiliary device 20 is not held by the holding device 40. Similarly, if the processor 250 does not detect that the second interface 230 is connected with the third interface 441 of the holding device 40 through the second interface 230, it determines that the monitoring auxiliary device 20 is not held by the holding device 40. The monitoring auxiliary device 20 being not held by the holding device 40, can be that for example, the monitoring auxiliary device 20 is not held by the holding device 40, or the monitoring auxiliary device 20 was previously held by the holding device 40 and later removed from the holding device 40. In this case, if the second interface 230 is not in electrical connection with the monitor 30 (such as wired connection), for example, the second interface 230 is not connected with the third interface 441, and the second interface 230 is not in wired connection with the monitor 30 via a cable, then the processor 250 can only transmit monitoring-related information to the monitor 30 through the wireless communication module 240. After a condition of a patient improves, the patient needs to get out of the patient support and move around. If the existing monitoring system is used, cables between sensors and the monitor need to be disconnected. After the patient has moved back to bed, all cables need to be re-connected, which is very complicated, and comprehensive monitoring cannot be carried out during the movement of the patient. By using the monitoring system provided by this disclosure, as shown in FIG. 8, it is only necessary to remove the monitoring auxiliary device 20 from the holding device 40 and allow the patient to carry it with them, even without plugging or unplugging cables, which is very convenient and can also monitor adequately during movement. When the patient returns to the patient support, only need to hold the monitoring auxiliary device 20 back by the holding device 40 to achieve wired connection with the monitor 30, which is very convenient. If the patient needs to be transferred, as shown in FIG. 9, the wired connection between the monitoring auxiliary device 20 and a bedside monitor can be disconnected. A small monitor (such as a transport monitor) can be held by a head of the patient support, and the small monitor can be wirelessly paired with the monitoring auxiliary device 20. The monitoring auxiliary device 20 wirelessly transmits monitoring related data to the small monitor in the above way, and the monitoring related data and corresponding monitoring results are displayed on a display interface of the small monitor, realizing monitoring of the patient by the small monitor. It can be seen that an operation of replacing the monitor and transferring the patient is convenient and fast. Of course, the bedside monitor can also be directly placed on the patient support for transportation. Due to a neat layout of the monitoring system cables in this disclosure, nurses do not need to disassemble the cables.

Of course, if the monitoring auxiliary device 20 is held by the holding device 40, but the second interface 230 is not in electrical connection with the monitor 30, for example, the second interface 230 is not connected with the third interface 441, and the second interface 230 is not in wired connection with the monitor 30 via a cable, then the processor 250 can only transmit monitoring-related information to the monitor 30 through the wireless communication module 240.

In some embodiments, as shown in FIG. 7, regardless of whether the monitoring auxiliary device 20 is held by the holding device 40, the monitoring auxiliary device 20 can be in direct electrical connection with the monitor 30, so as to obtain electrical power from the monitor 30 and transmit monitoring-related information to the monitor 30. That is, when the second interface 230 is in direct wired connection with the monitor 30 via a cable, the processor 250 outputs monitoring-related information through the second interface 230, and the monitoring-related information is transmitted to the monitor 30 through the cable. Electrical power can also be obtained from the monitor 30, through a power supply circuit formed by the second interface 230 and the cable, to the monitoring auxiliary device 20 for use. In order to ensure safety and stability of monitoring, when the processor 250 outputs monitoring-related information through the second interface 230, the processor 250 also transmits the monitoring-related information to the monitor 30 through the wireless communication module 240. The monitoring auxiliary device 20 can be used without cooperating with the holding device, but be directly connected with the monitor 30 via a cable. When the patient gets out of the patient support and moves around, not only does the monitoring auxiliary device 20 need to be not held by the holding device, but also the cable between the monitoring auxiliary device 20 and the monitor 30 needs to be disconnected. Compared with the previous embodiment, there is an additional operation of disconnecting the cable. When the monitoring auxiliary device 20 is not held by the holding device 40 or in wired connection with the monitor 30, the monitoring auxiliary device 20 obtains electrical power by the battery module 210 and transmits monitoring-related information to the monitor 30 through the wireless communication module 240.

The wireless communication module 240 can be a single wireless communication module, or can include more than one wireless communication modules, for example, including two wireless communication modules. This embodiment takes the latter as an example for explanation. The wireless communication module 240 includes a first wireless communication module and a second wireless communication module. The first wireless communication module is configured to wirelessly output monitoring-related information, and the second wireless communication module is also configured to wirelessly output monitoring-related information. The first wireless communication module and the second wireless communication module use different wireless communication manners. The different wireless communication manners can be that the two wireless communication modules use different communication technologies for communication, that is, communication protocols used by these two wireless communication modules are different, or that the two wireless communication modules use a same communication technology but different frequency bands for transmitting data or different frequencies for transmitting packets.

The processor 250 mentioned in the previous content transmits monitoring-related information to the monitor 30 through the wireless communication module 240, which can be done in various ways. Below are two examples for explanation.

In a wireless transmission method, the processor 250 transmits monitoring-related information to the monitor 30 through the first wireless communication module, and then transmits monitoring-related information to the monitor 30 through the second wireless communication module. Two wireless communication modules can simultaneously transmit the monitoring-related information to monitor 30, even if one wireless communication module has abnormal transmission, it will not affect monitoring.

Using this wireless transmission method, combined with the connection between the second interface 230 and the monitor 30 mentioned above, in an embodiment, when the second interface 230 is in wired connection with the monitor 30 (such as in direct wired connection with the monitor 30 or in wired connection with the monitor 30 via the holding device 40), the second interface 230 outputs monitoring-related information to the monitor 30 in a wired manner, and/or the first wireless communication module and/or the second wireless communication module transmit/transmits the monitoring-related information to the monitor 30. From a reliability perspective, when the second interface 230 is in wired connection with the monitor 30, at least the second interface 230 outputs monitoring-related information to the monitor 30 in a wired manner, and a wireless communication module still simultaneously transmits the monitoring-related information, or a wireless communication module maintains a wireless connection with the monitor. This helps to minimize data loss after the wired connection is disconnected. It can be understood that since a main application scenario of the monitoring auxiliary device in this scheme is in ICU, a continuity of transmission of monitoring-related information is very important. Once there is a loss of second(s) level, significant risks may be brought to patient monitoring. Accordingly, a simple manner of switching to wireless transmission after a disconnected wired connection takes some time, which has a significant impact in ICU scenarios. Therefore, this solution can choose to maintain wireless communication transmission or maintain wireless communication connection in wired transmission. When the second interface 230 is not in wired connection with the monitor 30, the first wireless communication module and the second wireless communication module simultaneously transmit monitoring-related information to the monitor 30.

In another wireless transmission method, when a preset condition is satisfied, the processor 250 transmits the monitoring-related information to the monitor 30 through the first wireless communication module. When a preset condition is not satisfied, the monitoring-related information is transmitted to the monitor 30 through the second wireless communication module. Alternatively, when a preset condition is not satisfied, the monitoring-related information is simultaneously transmitted to the monitor through both the first and second wireless communication modules. The preset condition includes a wireless communication connection between the first wireless communication module and the monitor 30 is reliable; and/or, the monitoring auxiliary device 20 is located within a preset range. That is, when the wireless communication connection between the first wireless communication module and the monitor 30 is unreliable, the processor 250 can switch to the second wireless communication module to transmit monitoring-related information (the first wireless communication module will be turned off after switching), or directly start the second wireless communication module to transmit monitoring-related information (the first wireless communication module will still be turned on). When the monitoring auxiliary device 20 is located outside the preset range, such as far away from the patient support, the processor 250 can switch to the second wireless communication module to transmit monitoring-related information, or directly start the second wireless communication module to transmit monitoring-related information. In this way, the reliability of wireless transmission of monitoring-related information can also be guaranteed. The processor 250 can determine the reliability of the wireless communication connection between the first wireless communication module and the monitor 30 based on a strength of a wireless signal between the first wireless communication module and the monitor 30. The processor 250 can also determine the reliability of the wireless communication connection between the first wireless communication module and the monitor 30 based on an interaction between the first wireless communication module and the monitor 30. For example, if the first wireless communication module fails to receive a response signal from the monitor 30 after transmitting data to the monitor 30, the wireless communication connection between the first wireless communication module and the monitor 30 is determined to be unreliable. A probability of packet loss between the first wireless communication module and the monitor 30 can also be used to determine the reliability of the wireless communication connection between the first wireless communication module and the monitor 30. Of course, these methods for determining the reliability of the wireless communication connection can also be executed by the monitor and fed back to the processor 250.

Using this wireless transmission method, combined with a connection situation between the second interface 230 and the monitor 30, in an embodiment, when the second interface 230 is in wired connection with the monitor 30, the second interface 230 outputs monitoring-related information to the monitor 30 in a wired manner, and/or the first wireless communication module and/or the second wireless communication module transmit/transmits monitoring-related information to the monitor 30.

When the second interface 230 is not in wired connection with the monitor 30 and a preset condition is satisfied, the processor 250 transmits monitoring-related information to the monitor 30 through the first wireless communication module.

When the second interface 230 is not in wired connection with the monitor 30 and a preset condition is not satisfied, the processor 250 can transmit monitoring-related information to the monitor 30 through the second wireless communication module, or transmit monitoring-related information to the monitor 30 through both the first and second wireless communication modules.

In this embodiment, when the first wireless communication module transmits monitoring-related information to the monitor 30, the first wireless communication module specifically uses WMTS (Wireless Medical Telemetry Service) technology to transmit monitoring-related information in a point-to-point wireless manner with the monitor 30, ensuring the security and reliability of data transmission. The second wireless communication module uses a Bluetooth module.

From the above content, it can be seen that this disclosure adds a monitoring auxiliary device and a holding device 40 to the traditional monitoring system, as shown in FIGS. 1 and 2. Through the monitoring auxiliary device 20, it is convenient for nurses to better manage cables on the patient support and for patients to get out of the patient support and move around. Through the monitoring auxiliary device and a holding device 40, monitoring-related information can be collected, as shown in FIG. 3, which includes the following steps.

Step 1, the monitoring auxiliary device 20 is in wired connection with multiple types of sensors 10, so as to obtain physiological parameter signals detected by the sensors 10. The specific process is described in the above embodiments and will not be elaborated here.

Step 2, when the monitoring auxiliary device 20 is held by the holding device 40, the monitoring auxiliary device 20 obtains electrical power via the holding device 40. When the monitoring auxiliary device 20 is held by the holding device 40, the monitoring-related information that is outputted by the second interface of the monitoring auxiliary device 20 is transmitted to the monitor via the holding device 40. The specific process is described in the above embodiments and will not be elaborated here.

Step 3, when the monitoring auxiliary device 20 is not held by the holding device 40 and does not output monitoring-related information in a wired manner, the monitoring auxiliary device 20 wirelessly outputs monitoring-related information to the monitor 30. The specific process is described in the above embodiments and will not be elaborated here.

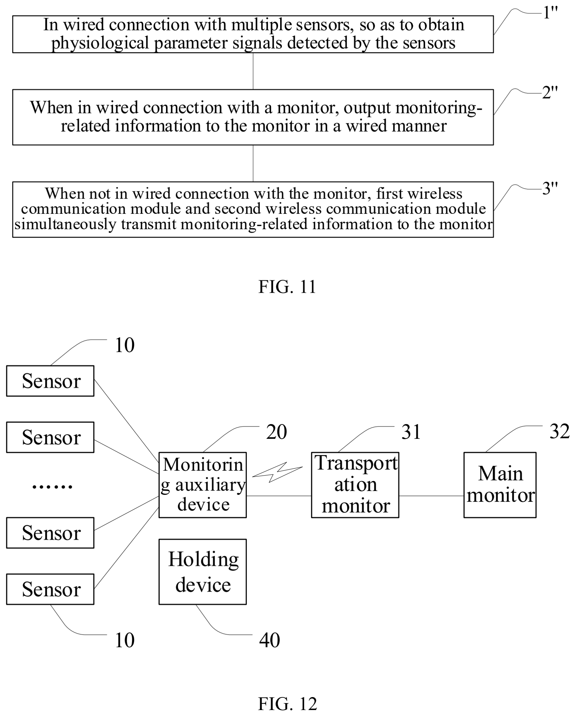

The method shown in FIG. 3 is explained from a position relationship between the monitoring auxiliary device 20 and the holding device 40, and can also be explained from a connection relationship between the monitoring auxiliary device 20 and the monitor 30, as shown in FIGS. 10 and 11, which will be introduced separately below.

As shown in FIG. 10, based on the monitoring system provided in the above embodiment, a method for collecting monitoring-related information includes following steps.

Step 1', the monitoring auxiliary device 20 is in wired connection with multiple types of sensors 10, so as to obtain physiological parameter signals detected by the sensors 10. The specific process is the same as step 1 above, and will not be repeated here.

Step 2' , when the monitoring auxiliary device 20 is in wired connection with the monitor 30, the monitoring auxiliary device 20 outputs monitoring-related information to the monitor 30 in a wired manner. The specific process is described in the above embodiments and will not be elaborated here.

Step 3', when the monitoring auxiliary device 20 is not in wired connection with the monitor 30, determine whether a preset condition is satisfied, if yes, proceed to step 4', if not, proceed to step 5'. The preset condition may include: a wireless communication connection between the first wireless communication module and the monitor 30 is reliable, and/or the monitoring auxiliary device 20 is located within a preset range.

Step 4', when the monitoring auxiliary device 20 is not in wired connection with the monitor 30 and a preset condition is satisfied, the first wireless communication module transmits monitoring-related information to the monitor 30. In this case, the second wireless communication module may not be turned on. The specific process is described in the above embodiments and will not be elaborated here.

Step 5', when the monitoring auxiliary device 20 is not in wired connection with the monitor 30 and a preset condition is not satisfied, the first wireless communication module stops transmitting monitoring-related information and the second wireless communication module is switched to transmit monitoring-related information to the monitor, or both the first and second wireless communication modules are switched to simultaneously transmit monitoring-related information to the monitor. The specific process is described in the above embodiments and will not be elaborated here.

In the embodiment shown in FIG. 11, in the monitoring system provided in the above embodiment, following steps for collecting monitoring-related information are included.

Step 1", the monitoring auxiliary device 20 is in wired connection with multiple sensors 10, so as to obtain physiological parameter signals detected by the sensors 10. The specific process is the same as step 1 above, and will not be repeated here.

Step 2", when the monitoring auxiliary device 20 is in wired connection with the monitor 30, the monitoring auxiliary device 20 outputs monitoring-related information to the monitor 30 in a wired manner. The specific process is described in the above embodiments and will not be elaborated here.

Step 3", when the monitoring auxiliary device 20 is not in wired connection with the monitor 30, the first wireless communication module and the second wireless communication module simultaneously transmit monitoring-related information to the monitor 30. The specific process is described in the above embodiments and will not be elaborated here.

### Second embodiment

The above-mentioned monitoring auxiliary device 20 can be appropriately simplified. In this embodiment, the monitoring auxiliary device 20 may not include the wireless communication module 240. That is, the monitoring auxiliary device 20 includes a first interface 220, a second interface 230 and a processor 250.

The first interface 220 is in wired connection with multiple types of sensors 10, so as to obtain physiological parameter signals detected by each sensor 10. The specific content is described in the above embodiments and will not be repeated here.

The second interface 230 can be used to obtain electrical power from an external power source. For example, when the monitoring auxiliary device 20 is held by the holding device 40, the second interface 230 obtains electrical power via the holding device 40. Specifically, when the monitoring auxiliary device 20 is held by the holding device 40 and the second interface 230 is connected with a third interface of the holding device 40, a power supply circuit formed by connecting the second interface 230, the third interface 441 and the cable L is configured to supply electrical power to the monitoring auxiliary device 20 from the monitor 30. The specific details are the same as the above embodiments and will not be repeated here.

The second interface 230 can also be configured to output monitoring-related information in a wired manner. For example, when the monitoring auxiliary device 20 is held by the holding device 40, the monitoring-related information that is outputted by the second interface 230 is transmitted to the monitor via the holding device 40. For example, when the monitoring auxiliary device 20 is held by the holding device 40 and the second interface 230 is connected with the third interface 441, the monitoring-related information that is outputted by the second interface 230 is transmitted to the monitor 30 through the power supply circuit. The specific details are the same as the above embodiments and will not be repeated here.

When the monitoring auxiliary device 20 is not held by the holding device 40 and is in electrical connection with the monitor 30 via a cable, the second interface 230 can obtain electrical power from the monitor 30 through the cable, and the monitoring-related information that is outputted by the second interface 230 can also be transmitted to the monitor 30 through the cable. That is to say, when the monitoring auxiliary device 20 is not used in conjunction with the holding device 40, the specific details are the same as the above embodiment and will not be repeated here.

The monitoring auxiliary device 20 of this embodiment may not include the battery module 210. As mentioned above, the monitoring auxiliary device 20 can obtain electrical power from the monitor 30 which is in wire connection, or wireless obtain electrical power from the holding device 40 through its own wireless charge-receiving module. The monitoring auxiliary device 20 is the same as the above embodiment except that it does not include the wireless communication module 240 and the battery module 210, and will not be repeated here.

### Third embodiment

The monitoring auxiliary device 20 of this embodiment includes not only the first interface 220, the second interface 230, and the processor 250 of the first embodiment, but also one or more wireless communication modules. The functions of the first interface 220, the second interface 230, and the wireless communication module have been detailed in the aforementioned embodiments and will not be repeated here.

The processor 250 can be configured to output monitoring-related information to the monitor 30 in a wired manner through the second interface 230, when the second interface 230 is in wired connection with the monitor 30, and configured to output monitoring-related information to the monitor 30 through the at least one wireless communication module, while the second interface 230 transmits the monitoring-related information through the wired connection. The second interface 230 being in wired connection with the monitor 30, as described in the above embodiments, can be that the second interface 230 is connected with the monitor through a third interface and a cable, or the second interface 230 is directly connected with the monitor via a cable. Please refer to the above embodiments for details, which will not be repeated here. The monitoring auxiliary device 20 simultaneously transmits monitoring-related information to the monitor through both wired and wireless manners, ensuring foolproof transmission of monitoring-related information.

The monitoring auxiliary device 20 including one or more wireless communication modules, can be including two or more wireless communication modules. This embodiment takes the former as an example to illustrate. The monitoring auxiliary device 20 includes a first wireless communication module and a second wireless communication module. The functions of the first wireless communication module and the second wireless communication module have been elaborated in detail in the aforementioned embodiments, and will not be repeated here.

When the processor 250 transmits monitoring-related information to the monitor 30 through wired communication on the second interface 230, at least one wireless communication module transmits the monitoring-related information to the monitor 30 by using following manners.

The processor 250 determines whether a communication quality of the first wireless communication module (such as a communication quality between the first wireless communication module and the monitor) satisfies a preset condition. For example, the processor 250 determines whether a wireless communication connection between the first wireless communication module and the monitor 30 is reliable. If yes, the processor 250 determines that the communication quality satisfies the preset condition. If not, the processor 250 determines that the communication quality does not satisfy the preset condition. Specifically, the processor 250 can determine whether the communication quality satisfies a preset condition based on a wireless signal strength between the first wireless communication module and the monitor 30. If the wireless signal strength is higher than a first preset threshold, the processor 250 determines that the communication quality satisfies a preset condition. If the wireless signal strength is lower than the first preset threshold, the processor 250 determines that the communication quality does not satisfy the preset condition. The processor 250 can also determine whether the communication quality between the first wireless communication module and the monitor 30 satisfies a preset condition through an interaction between the first wireless communication module and the monitor 30. If the first wireless communication module transmits data to the monitor 30 for one time and then does not receive a reply signal from the monitor 30, the processor 250 determines that the communication quality does not satisfy a preset condition. If a reply signal is received, the processor 250 determines that the communication quality satisfies the preset condition. The processor 250 can also determine whether the communication quality satisfies a preset condition based on a probability of packet loss between the first wireless communication module and the monitor 30. If the probability of packet loss is lower than a second preset threshold, the processor 250 determines that the communication quality satisfies a preset condition. If the probability of packet loss is higher than the second preset threshold, the processor 250 determines that the communication quality does not satisfy the preset condition. Of course, these methods for determining whether the communication quality satisfies a preset condition can also be executed by the monitor and a determination result can be fed back to the processor 250.

If the communication quality satisfies a preset condition, the processor 250 transmits the monitoring-related information to the monitor through the first wireless communication module, while transmitting the monitoring-related information through the second interface in a wired manner.

If the communication quality does not satisfy the preset condition, the processor 250 can stop a wireless communication of the first wireless communication module and transmit monitoring-related information through the second wireless communication module, while transmitting the monitoring-related information through the second interface in a wired manner. That is, the monitoring-related information will be transmitted to the monitor through the second wireless communication module, if a signal of the first wireless communication module is poor. Of course, when the communication quality does not satisfy the preset condition and the second interface transmits monitoring-related information in a wired manner, the processor 250 can also transmit monitoring-related information to the monitor simultaneously through the first wireless communication module and the second wireless communication module. In this way, it can ensure that the monitor can receive monitoring-related information.

The processor 250 can also output monitoring-related information to the monitor through the second interface in a wired manner when the second interface is connected with the monitor via a cable, and enable at least one wireless communication module to maintain a wireless communication connection with the monitor, while transmitting monitoring-related information through the second interface in a wired manner. When transmitting monitoring-related information through wired communication, at least one wireless communication module should maintain a wireless communication connection with the monitor, and be ready to transmit monitoring-related information at any time. In the event of a failure in wired transmission of monitoring-related information, the monitoring-related information can be immediately transmitted to the monitor through the at least one wireless communication module. Similarly, take at least one wireless communication module including a first wireless communication module and a second wireless communication module as an example. Transmitting, by the processor 250, monitoring-related information through the second interface in a wired manner, while maintaining wireless communication connection between at least one wireless communication module and the monitor, may include: determining, by the processor 250, whether the communication quality of the first wireless communication module satisfies a preset condition; if yes, transmitting monitoring-related information through the second interface in a wired manner while maintaining the first wireless communication module in a wireless communication connection with the monitor; if not, transmitting monitoring-related information through the second interface in a wired manner while maintaining the second wireless communication module in a wireless communication connection with the monitor or maintaining both the first wireless communication module and the second wireless communication module in a wireless communication connection with the monitor.

When the second interface is not in wired connection with the monitor, processor 250 can simultaneously transmit monitoring-related information to the monitor through the first wireless communication module and the second wireless communication module. The specific process has also been described in the previous embodiments and will not be repeated here.

### Fourth embodiment

The above-mentioned monitoring auxiliary device 20 can also be applied in transportation scenarios, as shown in FIG. 12. The monitoring system of this embodiment includes: a sensor 10, a monitoring auxiliary device 20, a transportation monitor 31, and a main monitor 32.

The function and function of sensor 10 are described in the above embodiments and will not be elaborated here.

The monitoring auxiliary device 20 is in wired connection with multiple sensors 10 to obtain physiological parameter signals detected by the sensors 10.

The monitoring auxiliary device 20 is configured to output monitoring-related information to the transport monitor 31 in a wired manner and/or a wireless manner, when the monitoring auxiliary device 20 is in wired connection with the transport monitor 31, usually the monitoring auxiliary device 20 is configured to at least output monitoring-related information to the transport monitor 31 in a wired manner.

The monitoring auxiliary device 20 is also configured to wirelessly output monitoring-related information to the transport monitor 31, when the monitoring auxiliary device 20 is not in wired connection with the transport monitor 31. The monitoring auxiliary device 20 is wirelessly pre-paired with the transport monitor 31. When the wired connection with the transport monitor 31 is disconnected or there is no wired connection, the monitoring-related information is transmitted to the transport monitor 31 through its own wireless communication module.

The transport monitor 31 is configured to receive the monitoring-related information outputted by the monitoring auxiliary device 20. For example, monitoring-related information is received by a corresponding communication interface in a wired transmission, while the monitoring-related information is received by a corresponding wireless communication module in a wireless transmission. In this embodiment, the transport monitor 31 can be used alone or in combination with the main monitor 32. The transport monitor 31 has a smaller volume than the main monitor 32, and a screen of the transport monitor 31 is also smaller than that of the main monitor 32. During regular use, the transport monitor 31 is inserted into a back of the main monitor 32 and fixed in place, achieving electrical connection between the transport monitor 31 and the main monitor 32, and integrating the transport monitor 31 and the main monitor 32 into one. Monitoring related data and monitoring results are displayed on the screen of the main monitor 32. In the transfer scenario, the transport monitor 31 can be separated from the main monitor 32 for separate use, making it easier to follow a patient during transfer. Therefore, the transport monitor 31 needs to determine its connection state with the main monitor 32. Specifically, the transport monitor 31 calculates a monitoring result based on at least part of the received monitoring-related information. The transport monitor 31 is also a type of monitor 30 in the above embodiment. Therefore, the connection relationship and data transmission between the monitoring auxiliary device, the holding device, and the monitor in the above embodiment are also applicable to the monitoring auxiliary device, the holding device, and the transport monitor. The specific process is described in the above embodiment and will not be repeated here. After receiving the monitoring-related information, the transport monitor 31 transmits the monitoring-related information or the monitoring results calculated based on the monitoring-related information to the main monitor. For example, the transport monitor 31 is coupled with the main monitor to achieve electrical connection with the main monitor. For example, the transport monitor 31 is plugged into the main monitor to achieve interface or contact docking that is adapted to the transport monitor 31 and the main monitor, forming an electrical connection. Through the electrical connection, monitoring-related information or monitoring results calculated based on monitoring-related information are transmitted to the main monitor. The transmission of monitoring-related information or monitoring results can be done in a wired manner or a wireless manner. For example, the transport monitor 31 determines whether it is in electrical connection with the main monitor 32. When the transport monitor 31 is not in electrical connection with the main monitor 32, the transport monitor 31 can output monitoring-related information and/or monitoring result(s) to the main monitor 32 or a central station in a wireless manner. This usually occurs during a transfer process, making it easier for nurses to remotely monitor patient through the main monitor 32 or the central station. In this case, the transport monitor 31 also displays monitoring-related information and/or monitoring result(s) on its own screen, making it easier for the nurse transferring the patient to monitor the patient. Please refer to FIGS. 2 and 9. When transportation is required, the nurse can directly remove a small transport monitor 31 from a large main monitor 32 and place the small transport monitor 31 next to the patient support. The entire process only requires the nurse to perform one removal operation. After pulling out the small transport monitor 31 and placing it next to the patient support, cables on a transfer patient support are simple and few, reducing maintenance time for nurses.

When the transport monitor 31 is in electrical connection with the main monitor 32, it outputs monitoring-related information and/or monitoring result(s) to the main monitor 32 in a wired manner, so that the main monitor 32 can display monitoring-related information and/or monitoring result(s). After being in electrical connection with the main monitor, the transport monitor 31 no longer displays monitoring-related information and monitoring result(s) calculated based on the monitoring-related information on its own screen. Instead, the main monitor displays, on its own screen, the monitoring-related information and/or monitoring result(s) calculated based on the monitoring-related information, after being in electrical connection with the transport monitor. It can be seen that when the transport monitor 31 is used in conjunction with the main monitor 32, if the transport monitor 31 is plugged into the main monitor 32, the transport monitor 31 and the main monitor 32 are integrated into one, equivalent to the monitor 30 in the above embodiment. The transport monitor 31 can achieve the data processing function of the monitor 30 in the first embodiment, and the main monitor 32 can achieve the display function of the monitor 30 in the first embodiment. The screen of the main monitor 32 is relatively large and mainly used for data display, which can achieve high human-machine interaction efficiency.

As shown in FIG. 4, the monitoring auxiliary device 20 can also include the battery module 210, the first interface 220, the second interface 230, the wireless communication module 240, and the processor 250 as described above. The monitoring auxiliary device 20 can also be directly used in conjunction with the transport monitor 31, and the specific content is the same as the case of directly connecting with the monitor 30 in the first embodiment. The monitoring auxiliary device 20 can also be used in conjunction with the holding device, and the latter will be used as an example for explanation.

The holding device 40 adapted to the monitoring auxiliary device 20 is detachably mounted on the patient support. When the monitoring auxiliary device 20 is held by the holding device 40, the monitoring auxiliary device 20 obtains electrical power via the holding device 40. For example, the monitoring auxiliary device 20 obtains electrical power via the holding device 40 through its own wireless charge-receiving module for use. For example, the monitoring auxiliary device 20 is connected with the holding device 40 through the second interface 230 and obtains electrical power from the holding device 40 through the second interface 230 for use. The specific implementation is the same as first embodiment, and will not be repeated here.

The monitoring-related information outputted from the second interface 230 of the monitoring auxiliary device 20 is transmitted to the transport monitor 31 via the holding device 40. For example, when the monitoring auxiliary device 20 is held by the holding device 40, the second interface 230 is connected with the third interface of the holding device 40, the monitoring auxiliary device 20 not only obtains electrical power from the transport monitor 31 through the power supply circuit formed by the second interface 230, the third interface, and the cable, but also transmits monitoring-related information to the transport monitor 31 through said power supply circuit. The specific implementation is the same as first embodiment, and will not be repeated here.

The monitoring-related information outputted by the wireless communication module 240 of the monitoring auxiliary device 20 can also be transmitted to the transport monitor 31 via the holding device 40, which is the same as the first embodiment and will not be repeated here.

When the monitoring auxiliary device 20 is not held by the holding device 40 and the second interface 230 does not output monitoring-related information, the wireless communication module 240 transmits the monitoring-related information to the transport monitor 31, which is the same as in the first embodiment and will not be repeated here.

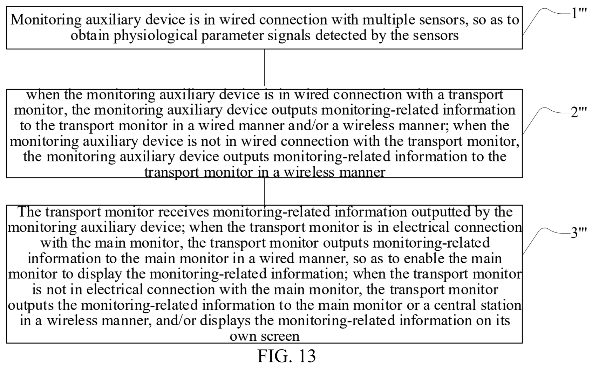

Based on the monitoring system provided in this embodiment, a collection process of monitoring-related information can be shown in FIG. 13, including following steps.

Step 1‴, the monitoring auxiliary device 20 is in wired connection with multiple sensors 10, so as to obtain physiological parameter signals detected by the sensors 10. The specific step 1 is the same as the above embodiment, and will not be repeated here.

Step 2"', when the monitoring auxiliary device 20 is in wired connection with the transport monitor 31, the monitoring auxiliary device 20 outputs monitoring-related information to the transport monitor 31 in a wired manner and/or a wireless manner; when the monitoring auxiliary device 20 is not in wired connection with the transport monitor 31, the monitoring auxiliary device 20 outputs monitoring-related information to the transport monitor 31 in a wireless manner. The specific process is described in the above embodiments and will not be elaborated here.

Step 3‴, the transport monitor receives monitoring-related information outputted by the monitoring auxiliary device; when the transport monitor is in electrical connection with the main monitor, the transport monitor outputs the monitoring-related information to the main monitor in a wired manner, so as to enable the main monitor to display the monitoring-related information; when the transport monitor is not in electrical connection with the main monitor, the transport monitor outputs the monitoring-related information to the main monitor or a central station in a wireless manner, and/or displays the monitoring-related information on its own screen. The specific process is described in the above embodiments and will not be elaborated here.

### Fifth embodiment

This embodiment is also applicable to a transportation scenario, and the difference from the fourth embodiment is that the monitoring auxiliary device 20 only transmits monitoring related data to the transportation monitor 31 in a wired manner, while the rest is the same as fourth embodiment. Specifically, the monitoring system provided in this embodiment includes: the sensor 10 as described above, the monitoring auxiliary device 20 as described above, the transport monitor 31 as described above, and the main monitor 32 as described above.

The monitoring auxiliary device 20 is in wired connection with multiple sensors 10, so as to obtain physiological parameter signals detected by the sensors 10.

The monitoring auxiliary device 20 is configured to output monitoring-related information to the transport monitor 31 in a wired manner, when the monitoring auxiliary device 20 is in wired connection with the transport monitor 31.

The transport monitor 31 is configured to receive monitoring-related information outputted by the monitoring auxiliary device 20, process at least part of the monitoring-related information, and obtain monitoring result(s). When the transport monitor 31 is in electrical connection with the main monitor 32, the transport monitor 31 outputs the monitoring-related information and/or monitoring result(s) to the main monitor 32 in a wired manner, so as to enable the main monitor 32 to display the monitoring-related information and/or monitoring result(s). The screen of the transport monitor 31 itself may not display the monitoring-related information and/or monitoring result(s). When the transport monitor 31 is not in electrical connection with the main monitor 32, the transport monitor 31 outputs the monitoring-related information and/or monitoring result(s) to the main monitor 32 or the central station in a wireless manner, and/or display the monitoring-related information and/or monitoring result(s) on its own screen.

The main monitor 32 is configured to receive and display the monitoring-related information and/or monitoring result(s) transmitted by the transport monitor 31.

Although an entire transmission process for physiological parameter signals, monitoring-related information and monitoring results is carried out through various interfaces and cables (wired), only one cable is needed between the monitoring auxiliary device 20 and the transport monitor 31. This makes it convenient for nurses to perform related operations in both conventional patient support rest and transport scenarios.

Technicians in this field can understand that all or part of functions of various methods in above embodiments can be implemented through hardware or computer programs. When all or part of functions in above embodiments are implemented through a computer program, the program can be stored in a computer-readable storage medium, which can include read-only memory, random access memory, magnetic disk, optical disk, hard disk, etc. The program can be executed by a computer to achieve above functions. For example, store the program in a device memory, when the program is executed by a processor, all or part of the above functions can be achieved. In addition, when all or part of the functions in the above embodiments are implemented through a computer program, the program can also be stored on a storage media, such as a server, another computer, disk, CD, flash drive, or portable hard drive. The program can be downloaded or copied to a memory of a local device, or a system of a local device can be updated in versions. When the program in the memory is executed by a processor, all or part of functions in above embodiments can be implemented.

This disclosure refers to various exemplary embodiments for explanation. However, technicians in this field will recognize that changes and modifications can be made to the exemplary embodiments without departing from the scope of this disclosure. For example, various operational steps and components used to perform them can be implemented in different ways based on specific applications or considering any number of cost functions associated with system operations (such as one or more steps being deleted, modified, or combined with other steps).

Furthermore, as understood by those skilled in the art, principles of this disclosure can be reflected in a computer program product on a computer-readable storage medium pre-mounted with computer-readable program instructions. Any tangible, non-temporary computer-readable storage medium can be used, including a magnetic storage device (a hard disk, a floppy disk, etc.), an optical storage device (CD-ROM, DVD, Blu Ray disk, etc.), a flash memory, and/or the likes. These computer program instructions can be loaded onto a general-purpose computer, a specialized computer, or another programmable data processing device to form a machine, so that the instructions executed on the computer or another programmable data processing device can generate a device that implements specified functions. These computer program instructions can also be stored in computer-readable memory, which can instruct a computer or other programmable data processing device to run in a specific manner, so as to enable the instructions stored in computer-readable memory to form a manufactured product, including implementation devices for implementing specified functions. Computer program instructions can also be loaded onto a computer or other programmable data processing device to perform a series of operational steps on the computer or other programmable device to generate a computer implemented process, such that instructions executed on the computer or other programmable device can provide steps for implementing specified functions.

Although the principles of this disclosure have been demonstrated in various embodiments, many modifications to structures, arrangements, proportions, elements, materials, and components that are particularly suitable for specific environments and operational requirements may be used without departing from the principles and scope of this disclosure. The above modifications and other changes or revisions will be included within the scope of this disclosure.

The foregoing detailed description has been described with reference to various embodiments. However, one skilled in the art will appreciate that various modifications and changes can be made without departing from the scope of this disclosure. Accordingly, this disclosure is to be considered in an illustrative rather than a restrictive sense, and all such modifications and changes are intended to be included within the scope thereof. Likewise, advantages, other advantages, and solutions to problems have been described above with regard to various embodiments. However, benefits, advantages, solutions to problems, and any elements that produce the same, or render them more evident, should not be construed as critical, required, or essential. As used herein, the term "includes," and any other variations thereof, are intended to be non-exclusive inclusions, such that a process, method, article, or apparatus that includes a list of elements includes not only those elements but also other elements that are not expressly listed or that do not belong to the process, method, system, article, or apparatus. Furthermore, the term "coupled" and any other variations thereof as used herein refers to a physical, electrical, magnetic, optical, communicative, functional, and/or any other connection.

Those skilled in the art will recognize that many changes can be made to the details of the above embodiments without departing from the basic principles of this disclosure. Therefore, the scope of this disclosure should be determined according to the following claims.

## Claims

1. A monitoring auxiliary device, **characterized in that**, comprising: one or more first interfaces, a second interface, a first wireless communication module and a second wireless communication module;
the one or more first interfaces are in wired connection with multiple types of sensors, so as to obtain physiological parameter signals detected by the sensors;
the second interface is connected with a monitor via a cable, so as to output monitoring-related information in a wired manner; wherein the monitoring-related information comprises the physiological parameter signals detected by the sensors, and/or physiological parameter data that are obtained based on processing the physiological parameter signals detected by the sensors;
the first wireless communication module is configured to output the monitoring-related information in a wireless manner; the second wireless communication module is also configured to output the monitoring-related information in a wireless manner; wherein the first wireless communication module and the second wireless communication module use different wireless communication manners;
when the second interface is not in wired connection with the monitor, the first wireless communication module and the second wireless communication module simultaneously transmit the monitoring-related information to the monitor.

2. A monitoring auxiliary device, **characterized in that**, comprising: a first interface, a second interface and one or more wireless communication modules;
the first interface is in wired connection with a sensor, so as to obtain a physiological parameter signal detected by the sensor;
the one or more wireless communication modules are configured to output monitoring-related information in a wireless manner; wherein the monitoring-related information comprises the physiological parameter signal detected by the sensor, and/or physiological parameter data that is obtained based on processing the physiological parameter signal;
when the second interface is in wired connection with a monitor, the second interface outputs the monitoring-related information to the monitor in a wired manner; wherein at least one of the one or more wireless communication modules transmits the monitoring-related information to the monitor, while the second interface outputs the monitoring-related information in a wired manner; or
when the second interface is connected with the monitor via a cable, the second interface outputs the monitoring-related information to the monitor in a wired manner; wherein at least one of the one or more wireless communication modules maintains a wireless communication connection with the monitor, while the second interface outputs the monitoring-related information in a wired manner.

3. The monitoring auxiliary device according to claim 2, **characterized in that**, the one or more wireless communication modules comprise a first wireless communication module and a second wireless communication module;
at least one of the one or more wireless communication modules transmitting the monitoring-related information to the monitor, while the second interface outputs the monitoring-related information in a wired manner, comprises:
determining whether a communication quality of the first wireless communication module satisfies a preset condition;
when the communication quality of the first wireless communication module satisfies the preset condition; transmitting the monitoring-related information to the monitor by the first wireless communication module, while the second interface outputs the monitoring-related information in a wired manner;
when the communication quality of the first wireless communication module does not satisfy the preset condition; transmitting the monitoring-related information to the monitor by the second wireless communication module, while the second interface outputs the monitoring-related information in a wired manner; or transmitting the monitoring-related information to the monitor by both the first wireless communication module and the second wireless communication module, while the second interface outputs the monitoring-related information in a wired manner.

4. The monitoring auxiliary device according to claim 2, **characterized in that**, when the second interface is not in wired connection with the monitor, the first wireless communication module and the second wireless communication module simultaneously transmit the monitoring-related information to the monitor.

5. A monitoring auxiliary device, **characterized in that**, comprising: a first interface, a second interface, a first wireless communication module and a second wireless communication module;
the first interface is in wired connection with a sensor, so as to obtain a physiological parameter signal detected by the sensor;
the second interface is connected with a monitor via a cable, so as to output monitoring-related information in a wired manner; wherein the monitoring-related information comprises the physiological parameter signal detected by the sensor, and/or physiological parameter data that is obtained based on processing the physiological parameter signal;
the first wireless communication module is configured to output the monitoring-related information in a wireless manner; the second wireless communication module is also configured to output the monitoring-related information in a wireless manner; wherein the first wireless communication module and the second wireless communication module use different wireless communication manners;
when the second interface is not in wired connection with the monitor and a preset condition is satisfied, the first wireless communication module transmits the monitoring-related information to the monitor;
when the second interface is not in wired connection with the monitor and the preset condition is not satisfied, the second wireless communication module transmits the monitoring-related information to the monitor, or the first wireless communication module and the second wireless communication module simultaneously transmit the monitoring-related information to the monitor;
wherein the preset condition comprises: a wireless communication connection between the first wireless communication module and the monitor is reliable, and/or the monitoring auxiliary device is located within a preset range.

6. The monitoring auxiliary device according to claim 5, **characterized in that**, when the second interface is in wired connection with the monitor; the second interface outputs the monitoring-related information to the monitor in a wired manner, and the first wireless communication module and/or the second wireless communication module output/outputs the monitoring-related information to the monitor in a wireless manner.

7. The monitoring auxiliary device according to claim 1, 2 or 5, **characterized in that**, the second interface is further configured to obtain electrical power from an external power source for the monitoring auxiliary device.

8. The monitoring auxiliary device according to claim 7, **characterized in that**, further comprising a battery module for supplying electrical power to the monitoring auxiliary device, when the external power source does not exist.

9. The monitoring auxiliary device according to claim 7, **characterized in that**, the monitoring auxiliary device is adapted to a holding device, wherein the holding device is detachably mounted on a patient support for holding the monitoring auxiliary device.

10. The monitoring auxiliary device according to claim 9, **characterized in that**, further comprising a wireless charge-receiving module, which is configured to obtain electrical power via the holding device for the monitoring auxiliary device.

11. The monitoring auxiliary device according to claim 9, **characterized in that**, the second interface configured to obtain electrical power from an external power source for the monitoring auxiliary device, comprises: the second interface configured to:
obtain the electrical power via the holding device for the monitoring auxiliary device, when the monitoring auxiliary device is held by the holding device.

12. The monitoring auxiliary device according to claim 11, **characterized in that**, the holding device comprises a third interface that is adapted to the second interface, wherein the third interface is in wired connection with the monitor via a cable;
the second interface configured to obtain the electrical power via the holding device for the monitoring auxiliary device, when the monitoring auxiliary device is held by the holding device, comprises: the second interface configured to:
obtain the electrical power from the monitor for the monitoring auxiliary device, via a circuit that is formed by connecting the third interface and said cable, when the monitoring auxiliary device is held by the holding device and the second interface is connected with the third interface.

13. The monitoring auxiliary device according to claim 12, **characterized in that**, the monitoring-related information that is outputted by the second interface is transmitted to the monitor via the holding device, when the monitoring auxiliary device is held by the holding device;
the monitoring-related information that is outputted by the second interface being transmitted to the monitor via the holding device, when the monitoring auxiliary device is held by the holding device, comprises:
the monitoring-related information that is outputted by the second interface is transmitted to the monitor via the circuit that is formed by connecting the third interface and said cable, when the monitoring auxiliary device is held by the holding device and the second interface is connected with the third interface.

14. The monitoring auxiliary device according to claim 8 or 9, **characterized in that**, the first wireless communication module and/or the second wireless communication module transmit/transmits the monitoring-related information to the monitor, when the monitoring auxiliary device is held by the holding device and the second interface is not connected with the third interface.

15. The monitoring auxiliary device according to claim 1, 2 or 5, **characterized in that**, when the second interface is in direct wired connection with the monitor via a cable, the monitoring-related information that is outputted by the second interface is transmitted to the monitor via said cable.

16. The monitoring auxiliary device according to claim 1, 3 or 5, **characterized in that**, the first wireless communication module transmitting the monitoring-related information to the monitor, comprises:
the first wireless communication module uses a WMTS technology to transmit the monitoring-related information to the monitor in a point-to-point wireless manner.

17. The monitoring auxiliary device according to claim 1, 3 or 5, **characterized in that**, the second wireless communication module is a Bluetooth module.

18. The monitoring auxiliary device according to claim 9, **characterized in that**, the first wireless communication module transmitting the monitoring-related information to the monitor, comprises:
after the first wireless communication module is in wireless connection with the holding device, the first wireless communication module transmits the monitoring-related information to the holding device in a wireless manner, so as to enable the holding device to transmit the monitoring-related information to the monitor.

19. The monitoring auxiliary device according to claim 1, 2 or 5, **characterized in that**, the first interface(s) is(are) in wired connection with multiple types of sensors; wherein one of the multiple types of sensors is a pressure sensor that is configured to detect a blood pressure signal;
wherein the monitoring auxiliary device further comprises:
a gas pump, which is configured to apply a pressure to a cuff of the pressure sensor, so as to enable the pressure sensor to detect the blood pressure signal.

20. The monitoring auxiliary device according to claim 1, 2 or 5, **characterized in that**, the first interface(s) is(are) in wired connection with multiple types of sensors; wherein the multiple types of sensors are configured to detect different physiological parameter signals, which comprise various types of: a blood oxygen signal, an electrocardiogram signal, a body temperature signal, a respiratory signal, and a blood pressure signal.

21. A monitoring auxiliary device, **characterized in that**, adapted to a holding device, wherein the holding device is detachably mounted on a patient support for holding the monitoring auxiliary device on the patient support; wherein the monitoring auxiliary device comprises:
a first interface, which is in wired connection with a sensor, so as to obtain a physiological parameter signal detected by the sensor;
a second interface, which is connected with a monitor via a cable and outputs monitoring-related information in a wired manner; wherein the monitoring-related information comprises the physiological parameter signal detected by the sensor, and/or physiological parameter data that is obtained based on processing the physiological parameter signal;
a wireless communication module, which is configured to output the monitoring-related information in a wireless manner;
when the monitoring auxiliary device is held by the holding device, the monitoring-related information that is outputted by the second interface is transmitted to the monitor via the holding device;
when the monitoring auxiliary device is not held by the holding device and the second interface does not output the monitoring-related information, the wireless communication module transmits the monitoring-related information to the monitor.

22. The monitoring auxiliary device according to claim 21, **characterized in that**, the second interface is further configured to obtain electrical power from an external power source for the monitoring auxiliary device.

23. The monitoring auxiliary device according to claim 22, **characterized in that**, further comprising a battery module for supplying electrical power to the monitoring auxiliary device, when the external power source does not exist.

24. The monitoring auxiliary device according to claim 21, **characterized in that**, further comprising a wireless charge-receiving module, which is configured to obtain the electrical power from the holding device for the monitoring auxiliary device.

25. The monitoring auxiliary device according to claim 22, **characterized in that**, the second interface configured to obtain electrical power from an external power source, comprises: the second interface configured to:
obtain the electrical power via the holding device, when the monitoring auxiliary device is held by the holding device.

26. The monitoring auxiliary device according to claim 25, **characterized in that**, the holding device comprises a third interface that is adapted to the second interface, wherein the third interface is in wired connection with the monitor via a cable;
the second interface configured to obtain the electrical power via the holding device, when the monitoring auxiliary device is held by the holding device, comprises: the second interface configured to:
obtain the electrical power from the monitor, via a circuit that is formed by connecting the third interface and said cable, when the monitoring auxiliary device is held by the holding device and the second interface is connected with the third interface.

27. The monitoring auxiliary device according to claim 26, **characterized in that**, the monitoring-related information that is outputted by the second interface is transmitted to the monitor via the holding device, when the monitoring auxiliary device is held by the holding device;
the monitoring-related information that is outputted by the second interface being transmitted to the monitor via the holding device, when the monitoring auxiliary device is held by the holding device, comprises:
the monitoring-related information that is outputted by the second interface is transmitted to the monitor, via the circuit that is formed by connecting the third interface and said cable, when the monitoring auxiliary device is held by the holding device and the second interface is connected with the third interface.

28. The monitoring auxiliary device according to claim 26 or 27, **characterized in that**, the wireless communication module transmits the monitoring-related information to the monitor, when the monitoring auxiliary device is held by the holding device and the second interface is not connected with the third interface.

29. The monitoring auxiliary device according to any one of claims 21-28, **characterized in that**, when the second interface is in direct wired connection with the monitor via a cable, the monitoring-related information that is outputted by the second interface is transmitted to the monitor via said cable.

30. The monitoring auxiliary device according to any one of claims 21-29, **characterized in that**, the wireless communication module comprises:
a first wireless communication module, which is configured to output the monitoring-related information in a wireless manner;
a second wireless communication module, which is also configured to output the monitoring-related information in a wireless manner;
wherein the first wireless communication module and the second wireless communication module use different wireless communication manners;
the wireless communication module transmitting the monitoring-related information to the monitor, comprises:
the first wireless communication module and the second wireless communication module both transmit the monitoring-related information to the monitor; or
the first wireless communication module transmits the monitoring-related information to the monitor; and when a preset condition is not satisfied, the second wireless communication module transmits the monitoring-related information to the monitor; wherein the preset condition comprises: a wireless communication connection between the first wireless communication module and the monitor is reliable, and/or the monitoring auxiliary device is located within a preset range.

31. The monitoring auxiliary device according to claim 30, **characterized in that**, the first wireless communication module transmitting the monitoring-related information to the monitor, comprises:
the first wireless communication module uses a WMTS technology to transmit the monitoring-related information to the monitor in a point-to-point wireless manner.

32. The monitoring auxiliary device according to any one of claims 21-30, **characterized in that**, the wireless communication module transmitting the monitoring-related information to the monitor, comprises:
after the wireless communication module is in wireless connection with the holding device, the wireless communication module transmits the monitoring-related information to the holding device in a wireless manner, so as to enable the holding device to transmit the monitoring-related information to the monitor.

33. The monitoring auxiliary device according to any one of claims 21-30, **characterized in that**:
when the monitoring auxiliary device is held by the holding device; while the monitoring-related information that is outputted by the second interface is transmitted to the monitor via the holding device, the wireless communication module simultaneously transmits the monitoring-related information to the monitor; and/or
when the second interface is in direct wired connection with the monitor via a cable, while the monitoring-related information that is outputted by the second interface is transmitted to the monitor via said cable, the wireless communication module simultaneously transmits the monitoring-related information to the monitor.

34. The monitoring auxiliary device according to any one of claims 21-33, **characterized in that**, the first interface is in wired connection with multiple types of sensors; wherein one of the multiple types of sensors is a pressure sensor that is configured to detect a blood pressure signal;
wherein the monitoring auxiliary device further comprises:
a gas pump, which is configured to apply a pressure to a cuff of the pressure sensor, so as to enable the pressure sensor to detect the blood pressure signal.

35. The monitoring auxiliary device according to any one of claims 21-33, **characterized in that**, the first interface is in wired connection with multiple types of sensors; wherein the multiple types of sensors are configured to detect different physiological parameter signals, which comprise various types of: a blood oxygen signal, an electrocardiogram signal, a body temperature signal, a respiratory signal, and a blood pressure signal.

36. A monitoring auxiliary device, **characterized in that**, adapted to a holding device, wherein the holding device is detachably mounted on a patient support for holding the monitoring auxiliary device on the patient support; wherein the monitoring auxiliary device comprises:
a first interface, which is in wired connection with a sensor, so as to obtain a physiological parameter signal detected by the sensor;
a second interface, which is connected with a monitor via a cable and outputs monitoring-related information in a wired manner; wherein the monitoring-related information comprises the physiological parameter signal detected by the sensor, and/or physiological parameter data that is obtained based on processing the physiological parameter signal;
when the monitoring auxiliary device is hold by the holding device, the monitoring-related information that is outputted by the second interface is transmitted to the monitor via the holding device.

37. The monitoring auxiliary device according to claim 36, **characterized in that**, the second interface is further configured to obtain electrical power from an external power source for the monitoring auxiliary device.

38. The monitoring auxiliary device according to claim 37, **characterized in that**, the second interface configured to obtain electrical power from an external power source, comprises: the second interface configured to:
obtain the electrical power via the holding device, when the monitoring auxiliary device is hold by the holding device.

39. The monitoring auxiliary device according to claim 38, **characterized in that**, the holding device comprises a third interface that is adapted to the second interface, wherein the third interface is in wired connection with the monitor via a cable;
the second interface configured to obtain the electrical power via the holding device, when the monitoring auxiliary device is held by the holding device, comprises: the second interface configured to:
obtain the electrical power from the monitor, via a circuit that is formed by connecting the third interface and said cable, when the monitoring auxiliary device is held by the holding device and the second interface is connected with the third interface.

40. The monitoring auxiliary device according to claim 39, **characterized in that**, the monitoring-related information that is outputted by the second interface is transmitted to the monitor via the holding device, when the monitoring auxiliary device is held by the holding device;
the monitoring-related information that is outputted by the second interface being transmitted to the monitor via the holding device, when the monitoring auxiliary device is held by the holding device, comprises:
the monitoring-related information that is outputted by the second interface is transmitted to the monitor, via the circuit that is formed by connecting the third interface and said cable, when the monitoring auxiliary device is held by the holding device and the second interface is connected with the third interface.

41. The monitoring auxiliary device according to any one of claims 36-40, **characterized in that**, when the monitoring auxiliary device is not held by the holding device and is in electrical connection with the monitor via a cable, the second interface obtains electrical power from the monitor via said cable, and/or the monitoring-related information that is outputted by the second interface is transmitted to the monitor via said cable.

42. A monitoring system, **characterized in that**, comprising: multiple sensors, a monitoring auxiliary device, a transport monitor and a main monitor;
the monitoring auxiliary device is in wired connection with the multiple sensors, so as to obtain physiological parameter signals detected by the multiple sensors;
the monitoring auxiliary device is configured to output monitoring-related information to the transport monitor in a wired manner and/or a wireless manner, when the monitoring auxiliary device is in wired connection with the transport monitor; the monitoring auxiliary device is further configured to output the monitoring-related information to the transport monitor in a wireless manner, when the monitoring auxiliary device is not in wired connection with the transport monitor; wherein the monitoring-related information comprises the physiological parameter signals detected by the sensors, and/or physiological parameter data that are obtained based on processing the physiological parameter signals;
the transport monitor is configured to receive the monitoring-related information that is outputted by the monitoring auxiliary device, and transmit to the main monitor the monitoring-related information or result(s) calculated based on the monitoring-related information.

43. The monitoring system according to claim 42, **characterized in that**, the transport monitor configured to transmit to the main monitor the monitoring-related information or result(s) calculated based on the monitoring-related information, comprises: the transport monitor configured to:
couple with the main monitor to achieve an electrical connection between the transport monitor and the main monitor; and
transmit to the main monitor, via the electrical connection, the monitoring-related information or the result(s) calculated based on the monitoring-related information.

44. The monitoring system according to claim 43, **characterized in that**,:
when the transport monitor is not in electrical connection with the main monitor, the transport monitor displays, on its own screen, the monitoring-related information or the result(s) calculated based on the monitoring-related information; after the transport monitor is in electrical connection with the main monitor, the transport monitor does not display, on its own screen, the monitoring-related information or the result(s) calculated based on the monitoring-related information;
after the main monitor is in electrical connection with the transport monitor, the main monitor displays, on its own screen, the monitoring-related information or the result(s) calculated based on the monitoring-related information.

45. The monitoring system according to claim 42, **characterized in that**, further comprising a holding device that is adapted to the monitoring auxiliary device;
wherein the holding device is detachably mounted on a patient support for holding the monitoring auxiliary device on the patient support;
wherein the monitoring auxiliary device comprises:
a second interface, which is connected with the transport monitor via a cable and outputs the monitoring-related information in a wired manner;
a wireless communication module, which is configured to output the monitoring-related information in a wireless manner;
a battery module, which is configured to supply electrical power to the monitoring auxiliary device when no external power source exists;
when the monitoring auxiliary device is held by the holding device, the monitoring-related information that is outputted by the second interface is transmitted to the transport monitor via the holding device;
when the monitoring auxiliary device is not held by the holding device and the second interface does not output the monitoring-related information, the wireless communication module transmits the monitoring-related information to the transport monitor.

46. The monitoring system according to claim 45, **characterized in that**, the monitoring auxiliary device further comprises a wireless charge-receiving module, which is configured to obtain electrical power via the holding device for the monitoring auxiliary device.

47. The monitoring system according to claim 42, **characterized in that**, the second interface is configured to obtain electrical power via the holding device, when the monitoring auxiliary device is held by the holding device.

48. The monitoring system according to claim 47, **characterized in that**, the holding device comprises a third interface that is adapted to the second interface, wherein the third interface is in wired connection with the transport monitor via a cable;
the second interface configured to obtain electrical power via the holding device, when the monitoring auxiliary device is held by the holding device, comprises: the second interface configured to:
obtain the electrical power from the transport monitor for the monitoring auxiliary device, via a circuit that is formed by connecting the third interface and said cable, when the monitoring auxiliary device is held by the holding device and the second interface is connected with the third interface.

49. The monitoring system according to claim 48, **characterized in that**, the monitoring-related information that is outputted by the second interface is transmitted to the transport monitor via the holding device, when the monitoring auxiliary device is held by the holding device;
the monitoring-related information that is outputted by the second interface being transmitted to the transport monitor via the holding device, when the monitoring auxiliary device is held by the holding device, comprises:
the monitoring-related information that is outputted by the second interface is transmitted to the transport monitor, via the circuit that is formed by connecting the third interface and said cable, when the monitoring auxiliary device is held by the holding device and the second interface is connected with the third interface.

50. A monitoring system, **characterized in that**, comprising: multiple sensors, a monitoring auxiliary device, a transport monitor, and a main monitor;
the monitoring auxiliary device is in wired connection with the multiple sensors, so as to obtain physiological parameter signals detected by the multiple sensors;
the monitoring auxiliary device is configured to output monitoring-related information to the transport monitor in a wired manner; wherein the monitoring-related information comprises the physiological parameter signals detected by the sensors, and/or physiological parameter data that are obtained based on processing the physiological parameter signals;
the transport monitor is configured to receive the monitoring-related information that is outputted by the monitoring auxiliary device, and output to the main monitor the monitoring-related information or result(s) calculated based on the monitoring-related information.
